# EUROPEAN PATENT SPECIFICATION

(11) **EP 3 038 592 B1**
(45) Date of publication and mention of the grant of the patent: **21.06.2017**
(21) Application number: 14741263.9
(22) Date of filing: 15.07.2014
(51) Int. Cl.: A61K 8/31, A61K 8/37, A61K 8/894, A61K 8/02, A61K 8/36, A61K 8/46, A61Q 19/10, A61K 8/92, A61K 8/04

(54) **FOAMABLE PERSONAL CARE COMPOSITION COMPRISING A CONTINUOUS OIL PHASE**
SCHÄUMBARE KÖRPERPFLEGEZUSAMMENSETZUNG MIT KONTINUIERLICHER ÖLPHASE
COMPOSITION MOUSSANTE DE SOINS PERSONNELS COMPRENANT UNE PHASE HUILEUSE CONTINUE

(30) Priority: 01.08.2013 US 201361861015 P
(43) Date of publication of application: 06.07.2016
(73) Proprietor: Unilever PLC, a company registered in England and Wales under company no. 41424 of, London EC4Y 0DY (GB); UNILEVER NV, 3013 AL Rotterdam (NL)
(72) Inventor: TSAUR, Liang Sheng, Trumbull, Connecticut 06611 (US)
(74) Representative: Fijnvandraat, Arnoldus
(86) International application number: PCT/EP2014/065102
(87) International publication number: WO 2015/014604

(56) References cited:
- WO-A1-2008/037609
- WO-A2-2005/058254
- WO-A2-2010/046354
- US-A1- 2012 094 885

## Description

This invention relates to foamable personal care compositions, including, for example, cleansing compositions, make-up removers, shaving compositions, and other lather-off compositions for skin and/or hair treatment, which compositions comprise a continuous oil phase containing dispersed solid synthetic cleansing surfactant and fatty acid soap crystals.

Many foamable personal care compositions are water-based liquids that include one or more synthetic cleansing, i.e., detersive, surfactants commonly known as "syndet" and herein alternatively referred to as "synthetic surfactant" or "cleansing surfactant". The compositions, commonly oil-in-water emulsions, typically require production techniques that employ one or more heating steps in which a surfactant-containing aqueous phase is heated to temperatures on the order of 50-90°C, followed by cooling and subsequent combination with an oil phase and other composition ingredients, e.g., volatiles. Heating and subsequent cooling can add to production costs and equipment requirements. Such requirements can also become time limiting steps during production. Additionally, creating concentrates with conventional water-based liquid detergents can be problematic in that the reduced amount of water in a concentrate can give rise to products that are prone to gel formation and, as a result, are too viscous to be easily applied or diluted.

Fluid compositions having a continuous oil phase offer potential advantages over conventional water-based compositions in one or more of the aforesaid areas, i.e., processing, concentrate formation, and so forth.

Certain oil-based personal care compositions have been suggested:

US 4,673,526 to Zabotto et al. describes an anhydrous skin cleansing composition containing an oil phase, emulsifying agent and water soluble polymeric abrasive particles.

US 8,063,005 to Kalidini discloses personal care formulations said to have simultaneous exfoliating, cleansing and moisturizing properties, which formulations comprise an oil phase and a natural surfactant derived from legumes and/or grains.

US 6,620,773 to Stork et al. discloses foaming oil preparations that include clear single-phase foaming oil formulations containing a mixture of oil and specific surfactants.

US 5,653,988 to Gerber et al. discloses substantially anhydrous clear shower oil composition containing up to 55% by weight of an amide of a fatty alcohol sulfate or an amide of a fatty alcohol ether sulfate.

US 6,524,594 to Santora et al. discloses a gelled oil skin cleansing composition comprising 3 to 10%, based on the total weight of the composition, of a gelling agent selected from dextrin myristate or a blend of at least two different polymers consisting of diblock or triblock copolymers. The patent further discloses that the gelled composition is "essentially water-free", therein understood to mean that based on the total weight thereof, the gelled composition contains no more than about 5 percent of water, and preferably comprises no more than about 3 percent of water.

US 2005/0158351A1 to Soliman *et. a*/*.* discloses an anhydrous ex-foliative foaming cleanser containing (1) 5 to 40wt.% of large ex-foliative particles having a mean particle size of 50 to 1200 microns to serve as scrubbing agent against the skin; (2) 0.5 to 15 wt.% anionic surfactants; (3) emollient oil and at least one oil gelling agent for phase stability. Exemplary of such gelling agents are silica, clays and organically modified clays and mixtures thereof. WO2008/037609 relates to a liquid cleansing composition that contains high levels of hydrocarbon wax and oil emollients yet produces substantial levels of foaming. The cleansing composition contains C6 to 20 acyl sarcosinate surfactant(s) and total hydrocarbon wax and oil emollients in a specific ratio range and preferably has a liquid crystalline structure.

WO 2010/046354 relates to soap-based liquid body and facial wash compositions. Using high solvent, low water compositions and incompletely naturalized fatty acids to help structure the compositions, all in combination with modified benefit agents, enhanced deposition of various agents is achieved.

Notwithstanding the foregoing, formulating continuous oil phase compositions with desirable phase stability has been problematic. When present as the disperse phase of an oil-based composition, solid surfactant particles tend to separate over time, forming what can be a dense layer of the particles at the bottom of the composition and an oil layer on the top. The tendency toward particle separation is increased with larger and/or heavier particles, i.e., particles having a weight average particle size of greater than 50µm. Conventional routes to stabilization, e.g., the addition of viscosity modifiers and thickening agents, can interfere with surfactant release in use, impeding foam formation and lathering.

There remains a need for foamable personal care compositions, in particular, fluid compositions that can be produced under conditions that minimize the need to employ heating and cooling and, if desired, that can be formulated over a broad range of solid synthetic cleansing surfactant concentrations, for example, up to 65% by weight, more particularly 20 to 60% by weight, based on the total weight of the composition. There remains a further need for foamable fluid personal care compositions having a continuous oil phase (i.e., oil-continuous compositions), and, more particularly, for storage stable oil-continuous compositions that, when foamed, have good lather properties.

It has now been found that foamable fluid compositions comprising a continuous oil phase in which is dispersed solid synthetic surfactant, can be stabilized through the inclusion in such oil phase of a relatively small amount of fatty acid soap crystals, i.e., 0.5 to 10% by weight, more particularly 1 to 8% by weight, even more particularly 1 to 5% by weight, based on the total weight of the composition.

The subject invention provides a product format that offers convenient preparation for personal care compositions, in particular, the ability to achieve, if desired, relatively high loadings of solid synthetic cleansing surfactant in a composition that affords good phase stability, that is to say, the structure of the composition is less susceptible to collapsing and the surfactant is less susceptible to sedimentation.

Without wishing to be bound to theory, it is believed that the fatty acid soap crystals aid in forming a microstructure that improves the storage stability of the fluid composition. Additionally, the fatty acid soap crystals can increase composition viscosity without giving rise to undesirable gelation, and provide a thickening mechanism that gives rises to desirable release of the solid synthetic cleansing surfactant in use. In turn, the surfactant release properties aid in the production of creamy, thick foams.

Pursuant to the subject invention there is provided a foamable, fluid personal care composition comprising a continuous oil phase in which is dispersed fatty acid soap crystals and solid synthetic cleansing surfactant wherein the composition comprises:
i. 35 to 90% by weight, preferably 35 to 80% by weight, preferably 40 to 80% by weight, of carrier oil;
ii. 0.5 to 10% by weight, preferably 1 to 8% by weight, of fatty acid soap crystals; and
iii. 10 to 65% by weight, preferably 10 to 60% by weight, preferably 20 to 60% by weight, of solid synthetic cleansing surfactant;
wherein fatty acid soap means salt of aliphatic alkane or alkene monocarboxylic fatty acids having 6 to 22 carbon atoms; and
wherein the solid synthetic cleansing surfactant is present in the form of particles of size within a range of from 0.5 to 2000µm, wherein at least 50% by weight of the particles are less than 200µm in size and at least 30% by weight of the particles are less than 150µm in size. In use, the composition is diluted with water, dissolving and releasing the surfactant and generating lather.

As used herein, the term "fluid" refers to a composition that at 1 atmosphere (101,325 Pa) and 23°C has a viscosity within a range of 300 to 50,000 kgm⁻¹s⁻¹ (i.e. Pa.s) at 0.01 rps (reciprocal second) at 23°C. Owing to the way it is stabilized, the oil-continuous composition of the subject invention exhibits shear thinning rheology, i.e., viscosity decreases with increasing shear rate. Typically, the compositions of this invention have a viscosity at 0.01 rps at and 23°C that is least 10 times greater than the viscosity thereof at 1 rps and 23°C. In one or more preferred embodiments, the viscosity of the compositions at 1 rps and 23°C is within a range of 10 to 500 kgm⁻¹s⁻¹. Viscosity may be conveniently measured using a standard AR-G2 stress-controlled rheometer from Texas Instruments (or equivalent) and following the viscosity measurement procedure described in the Examples that follow. The viscosity range of interest encompasses a broad range of pourable and/or pumpable compositions including liquid, pastes, and creams.
The oil phase of the subject composition comprises one or more cosmetically acceptable carrier oils. Among the carrier oils suitable for use herein are natural and synthetic oils that are liquid at 1 atmosphere (101,325 Pa) and 25°C. Included among such oils are hydrocarbon oils, ether oils, ester oils, fatty alcohols, and silicone oils.
Among the suitable hydrocarbon oils are linear, branched, and cyclic oils, non-limiting examples of which include, for example, liquid paraffin, squalene, squalane, mineral oil, low viscosity synthetic hydrocarbons such as, for example, polyalphaolefin sold by ExxonMobil under the trade name of PureSyn™POA, polybutene, including oils available from INEOS under the trade name of PANALANE^{®} or INDOPOLE^{®}, and diethyl hexyl cyclohexane. Light (low viscosity) highly branched hydrocarbon oils are also suitable.
Among the suitable ester oils are, for example, mono- and polyfunctional esters such as cetyl octanoate, octyl isonanoate, myristyl lactate, cetyl lactate, cetyl ethyl hexanoate, isopropyl myristate, myristyl myristate, isopropyl palmitate, isopropyl adipate, diisopropyl adipate, isopropyl isostearate, butyl stearate, decyl oleate, isodecyl oleate, glycerol monostearate, glycerol distearate, glycol tristearate, and glyceryl tri (2-ethylhexanoate); as well as modified ester oils such as, for example, alkoxylated esters including, for example PPG-3-benzyl ether myristate, and the like. Other suitable ester oils are triglycerides and modified triglycerides. These include vegetable oils such as, for example, jojoba, safflower, sunflower seed, palm kernel, soybean, castor, coconut, olive, rice-germ, sweet almond, rapeseed, wheat-germ, and grape seed oil, and the like. Synthetic triglycerides may also be employed. Among the modified triglycerides are materials such as, for example ethoxylated and maleated triglyceride oils. Among the triglyceride oils, materials of particular interest include C₈-C₁₈ and especially the C₈-C₁₂, fatty acid triglycerides. Proprietary ester blends such as those sold by Finetex under the trade name Finsolv^{®}, may also be employed herein. Another type of suitable ester is liquid polyester formed from the reaction of a dicarboxylic acid and a diol including, for example, the polyester marketed by ExxonMobil under the trade name PureSyn™ Ester.
Ether oils suitable for use herein include, for example, polyglycols (especially from polypropylene glycol (PPG) containing at least 3 mers, such as 3 to 20) of monohydric alcohols (the monohydric alcohols often containing between 3 and 20 carbons). As the molecular weight of the PPG increases, the chain length of the monohydric alcohol can decrease. For example, suitable ether oils can vary between a low molecular weight PPG with a relatively long chain fatty alcohol (an example of such an oil being PPG-3 myristyl ether), and a lower alkyl ether of a higher molecular weight PPG (an example of such an oil being PPG-14 butyl ether).
The alcohols suitable for use herein, include, for example, oleyl alcohol, isostearyl alcohol, hexyl decanol, 2-octyldodecanol (Eutanol^{®} G) and the like.
Among the suitable silicone oils are polysiloxanes, including, for example, linear and cyclic polydimethylsiloxanes, as well as organofunctional silicones (alkyl and alkyl aryl), and hydroxyl- and amino- silicones.
The carrier oils may be used individually or as combinations of two or more oils from the same and/or different classes. Of particular interest as carrier oils are hydrocarbon oils (mineral oil in particular), triglyceride oils and silicone oils.
In one or more embodiments, all or substantially all, that is to say up to 90% by weight, preferably up to 95% by weight, more preferably up to 97% by weight of the carrier oil is non-volatile. Preferably, the non-volatile oil has a vapor pressure less than 0.5 mm Hg (66 Pa) at 23°C. From a material handling perspective, the non-volatile oil preferably has a boiling point in excess of 100°C, more preferably in excess of 150°C. In other embodiments it is contemplated that up to 30 wt.%, more particularly up to 15 wt.%, of the carrier oil is volatile oil. Replacement of a portion of the non-volatile carrier oil with volatile carrier oil may provide certain sensory advantages.
In the subject compositions carrier oil is present in an amount of 35 to 90% by weight, preferably 35 to 80 % by weight, preferably 40 to 80% by weight, based on the total weight of the composition. In at least one embodiment of particular interest, 50 to 100% by weight, more particularly 70 to 100% by weight, even more particularly 90 to 100% by weight of the total carrier oil is provided by one or more non-volatile oils, the non-volatile oils preferably being selected from hydrocarbon oils, triglyceride oils, and silicone oils. In use, the carrier oil helps to impart a smooth and moisturizing skin feel.

As used herein reference to "carried oil" is exclusive of fragrance and the class of plant derived materials commonly known as essential oils.

The term "fatty acid soap" or, more simply, "soap", "soap salt" or "neutralized fatty acid" is used here in its popular sense, i.e., salts of aliphatic alkane- or alkene monocarboxylic fatty acids preferably having 6 to 22 carbon atoms and, more preferably, 8 to 18 carbon atoms. Typical of the soap salts are alkali metal (e.g., potassium and sodium) and alkanol ammonium (e.g., mono-, di- and triethanol ammonium) salts of such fatty acids, although other metal salts thereof, e.g., magnesium salts, may also be employed. Commonly, potassium and/or sodium soaps are used in the formulations of this invention.

Fatty acid soap is distinguished from and not be considered as being among the materials herein referred to as synthetic cleansing surfactant. Preferably, the fatty acid soap has a melting point in the presence of the carrier oil in excess of 40°C, preferably in excess of 45°C, and most preferably in excess of 50°C. The fatty acid soap preferably has a melting point that is high enough to withstand the storage temperatures contemplated for the resulting composition.

The fatty acids from which the soap salts are derived may contain unsaturation, however, excessive unsaturation is normally avoided to minimize color and odor issues. Additionally, excessive unsaturation can give rise to reduced melting points and can interfere with the formation of crystal structure. Commonly, not more than 30 wt.% of the fatty acids from which the soap salts are formed are unsaturated.

In one or more embodiments of interest, the combination of C₁₂, C₁₄, C₁₆, and C₁₈ fatty acids constitutes 90 to 100 wt%, more particularly 95 to 100 wt.% of the total fatty acids from which the soap crystals are formed and, preferably, the combination of C₁₆ and C₁₈ fatty acids constitutes 10 to 35 wt.%, more particularly 10 to 30 wt% of the total fatty acids from which the soap crystals are formed. In one or more embodiments, C₁₂ fatty acid constitutes 60 to 80 wt.% of the total fatty acids from which the soap crystals are formed. Preferably, the total amount of C₈ and C₁₀ fatty acids is less than 5 wt%, and preferably is less than 3 wt.% of the total fatty acids from which the soap crystals are formed.

To form fatty soap crystals with desirably high melting points, it is generally preferred that the fatty acid soap is at least 70%, preferably at least 80% neutralized. If excessively over-neutralized, the soap has the potential of hydrolyzing the oil component. In one or more embodiments of interest, the fatty acid soap has a degree of neutralization of 70 to 100%, more particularly, 80 to 100%, and even more particularly, 80 to 95%.

Reference to fatty acid soap "crystals" means that the fatty acid soap is in crystalline form, preferably as elonged or needle- or rod-like structures. Commonly, at least 95% by weight, more particularly at least 97% by weight, of the soap crystals are of length less than 300µm and, preferably, at least 90% by weight of the soap crystals are less than 200µm and, more preferably, are less than 100µm in length. In one or more preferred embodiments, at least 40% by weight of the soap crystals are of length within a range of 5 to 100µm, and more particularly are of length within a range of 5 to 50µm.

To provide the subject compositions with fatty acid soap in crystal form it is preferable to introduce same as pre-made crystals as, for example, by combining the starting fatty acids with a carrier oil, melting same to form a solution or mixture of the molten fatty acid in oil, and, under shear, adding neutralizing agent and cooling to generate a dispersion of soap crystals in oil, which dispersion may be used as an ingredient in the preparation of the subject compositions. In one or more embodiments, such a dispersion typically contains 10 to 50% by weight, more particularly 20 to 40% by weight of soap crystals. Employing dispersions with less than 10% by weight of soap crystals is inefficient from a material handling and cost perspective; conversely, obtaining in excess of 50% by weight of soap crystals in the dispersion is uncommon when fatty acids are employed as starting materials.

The fatty acid soap crystals are present the subject compositions in an amount of 0.5 to10% by weight, based on the total weight of the composition. Preferably, the fatty acid soap crystals are present in an amount of 1 to 8% by weight, based on the total weight of the composition. In one or more embodiments of interest, fatty acid soap crystals are present in an amount of 2 to 8% by weight, more particularly, 2 to 6% by weight, based on the total weight of the composition.

The solid synthetic cleansing surfactant has a melting point above 25°C, preferably above 35°C, and more preferably above 40°C. In one or more embodiments, cleansing surfactant having a melting point above 50°C is of particular interest. Commonly, the solid synthetic cleansing surfactant includes one or more surfactants selected from anionic surfactant, zwitterionic surfactant, amphoteric surfactant, and mixtures thereof. In the surfactant descriptions that follow, carbon contents ought to be understood as modified by the word "about".

The anionic surfactant may be, for example, an aliphatic sulfonate, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGES); or an aromatic sulfonate such as alkyl benzene sulfonate. The anionic surfactant may also be an alkyl sulfate (e.g., C₈-C₂₂ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 22 carbons, preferably 8 to 18 carbons, more preferably 12 to 18 carbons; n has an average value of greater than 1.0, preferably between 2 and 3; and M is a solubilizing cation such as, for example, sodium, potassium, ammonium or substituted ammonium. Ammonium and sodium laurel ether sulfates are preferred. The anionic surfactant may also be alkyl sulfosuccinates (including mono- and dialkyl, e.g., C₈-C₂₂ sulfosuccinates); alkyl and acyl taurates, alkyl and acyl sarcosinates, sulfoacetates, C₈-C₂₂ alkyl phosphates and phosphates, alkyl phosphate esters and alkoxyl alkyl phosphate esters, acyl lactates, C₈-C₂₂ monoalkyl succinates and maleates, sulphoacetates, acyl isethionates, and acyl glycinates.

Sulfosuccinates may be monoalkyl sulfosuccinates having the formula:

R¹O₂CCH₂(SO₃M)CO₂M;

and amido-MEA sulfosuccinates of the formula:

R¹CONHCH₂CH₂O₂CCH₂CH(SO₃M)CO₂M ;

wherein in such formulas R1 ranges from C₈-C₂₂ alkyl and M is a solubilizing cation as described above; or amido-MIPA sulfosuccinates of formula:

RCONH(CH₂)CH(CH₃)(SO₃M)CO₂M

wherein R and M are as described above. Also included are the alkoxylated citrate sulfosuccinates; and alkoxylated sulfosuccinates such as the following:

R-O-(CH₂CH₂O)ₘ-C(O)-CH₂CH(SO₃M)CO₂M)

wherein m=1 to 20; and R and M are as described above.

Sarcosinates are generally indicated by the formula R²CON(CH₃)CH₂CO₂M, wherein R² ranges from C₈-C₂₀ alkyl and M is a solubilizing cation.

Taurates are generally identified by formula:

R²CONR³CH₂CH₂SO₃M

wherein R² is described above, R³ ranges from C₁-C₄ alkyl and M is a solubilizing cation.

Another class of anionic cleansing surfactants are carboxylates such as follows:

R²(CH₂CH₂O)ₚM

wherein R² is as described above, p is 0 to 20, and M is a solubilizing cation. Yet another carboxylate which can be used is amido alkyl polypeptide carboxylates such as, for example, is available from Seppic under the trademark Monteine^{®}.

Yet another class of cleansing surfactants which may be used herein are the acyl isethionates, including, for example, the C₆-C₂₂ acyl isothienates. These esters are commonly prepared by reaction between alkali metal isethionate with mixed aliphatic fatty acids having from 6 to 22 carbon atoms and an iodine value of less than 20, preferably at least 75% of the mixed fatty acids have from 12 to 18 carbon atoms. The acyl isethionate may be an alkoxylated isethionate such as is described in Ilardi et al., U.S. Pat. No. 5,393,466.

Acyl glycinates are representative of yet another class of suitable anionic cleansing surfactant. The acyl glycinates are generally represented by the formula:

R²CONHCH₂COOM

where R² and M are as described above. Suitable acyl glycinates include for example, sodium cocoyl glycinate, sodium stearoyl glycinate, sodium lauroyl glycinate, sodium lauroyl sarcosinate, and potassium cocoyl glycinate.

In general, anionic cleansing surfactant will comprise 0 to 100% by weight, preferably 30 to 100% by weight, most preferably 50 to 85% by weight or more of the total solid cleansing surfactant.

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents has from 8 to 22 carbon atoms and one contains an anionic group, e.g., carboxy, sulfonate, sulfate, phosphate, or phosphonate. A general formula for many of these compounds is: 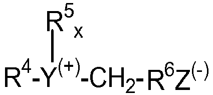 wherein R⁴ contains an alkyl, alkenyl, or hydroxy alkyl radical of from 8 to 22 carbon atoms, from 0 to about 10 ethylene oxide moieties and from 0 to about 1 glyceryl moiety; Y is selected from the group consisting of nitrogen, sulfur and phosphorous atoms; R⁵ is an alkyl or monohydroxyalkyl group of from 1 to 3 carbon atoms; x is 1 when Y is a sulfur atom, and 2 when Y is a nitrogen or phosphorus atom; R⁶ is an alkylene or hydroxyalkylene of from 1 to 4 carbon atoms and Z is a radical selected from the group consisting of carboxylate, sulfonate, sulfate, phosphonate, and phosphate groups.

Amphoteric cleansing surfactants which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 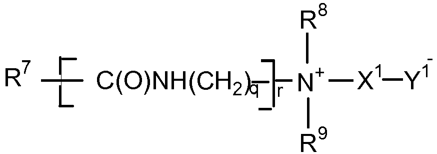 where R⁷ is alkyl or alkenyl of 7 to 18 carbon atoms; R⁸ and R⁹ are each independently alkyl, hydroxyalkyl or carboxyalkyl of 1 to 3 carbon atoms; q is 2 to 4; r is 0 to 1; X¹ is alkylene of 1 to 3 carbon atoms optionally substituted with hydroxyl, and Y¹ is -CO₂- or -SO₃-.

Suitable amphoteric cleansing surfactant within the above general formula include simple betaines of formula: 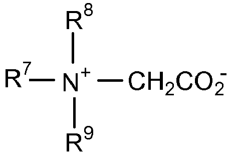 and amido betaines of formula: 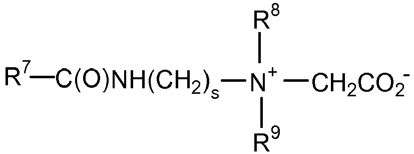 where s is 2 or 3. In both formulae R⁷, R⁸ and R⁹ are as previously described R⁷ may, in particular, be a mixture of alkyl groups derived from coconut so that at least half, preferably at least three quarters of the groups R⁷ have 10 to 14 carbon atoms. Preferably, R⁸ and R⁹ are methyl.

A further possibility is that the amphoteric cleansing surfactant is a sulphobetaine of formula: 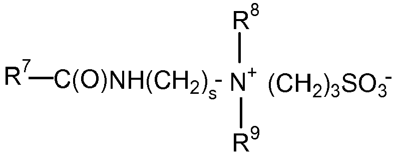 where s is as previously described, and preferably is 2, or variants of these in which -(CH₂)₃ SO₃⁻ is replaced by -CH₂C(OH)HCH₂SO₃⁻. In these formulae R⁷, R⁸ and R⁹ are as discussed previously.

Amphoacetates and diamphoacetates are also intended to be covered in possible zwitterionic and/or amphoteric compounds which may be used.
Together, zwitterionic and amphoteric surfactant may comprise from 0 to 100% by weight of the total solid cleansing surfactant and, when present, will preferably comprise 10 to 60% by weight, more preferably from 10 to 40% by weight, of the total solid cleansing surfactant. In one or more preferred embodiments, together, anionic and amphoteric surfactant comprise 80 to 100% and, more particularly, 90 to 100% by weight of the total solid cleansing surfactant.
The composition may optionally, though less commonly, comprise other types of solid cleansing surfactant, e.g., nonionic and cationic surfactant. If present the total amount thereof is typically, 0.1 to 40% by weight, more particularly 0.5 to 10% by weight of the total solid cleansing surfactant.
In one or more embodiments, compositions having relatively high levels of solid cleansing surfactant, for example, 35 to 65% by weight and, more particularly, 40 to 60% by weight, based on the total weight of the composition, are of interest. In at least one embodiment, the composition comprises 35 to 55% by weight of solid cleansing surfactant, based on the total weight of the composition.
The solid cleansing surfactant is present in the subject composition in the form of dispersed particles. As used herein, all references to "size" and "size distribution" of solid cleansing surfactant particles are to the particles as primary particles; where the particles are in aggregated form, these parameters given in reference to the particles that make-up the aggregate, as opposed to the aggregate made up of the particles. The size of the solid surfactant particles is within a range 0.5 to 2000µm, wherein at least 50% by weight, preferably at least 60% by weight, more preferably at least 65% by weight of the particles are less than 200 µm in size and at least 30% by weight, preferably at least 40% by weight, most preferably at least 50% by weight of the particles are less than 150 µm in size. Preferably no more than 45% by weight, more preferably no more than 30% by weight of the particles are greater than 500µm in size. In reference to a particle, size refers to the particle's largest diameter, as a primary particle.
In addition to impacting product stability, viscosity, and appearance, the size and size distribution of the solid cleansing surfactant particles can also affect in-use properties such as speed to lather and product "feel". In at least one embodiment, a desirable combination of properties is provided by the particles of solid cleansing surfactant having a size distribution wherein at least 40% by weight, preferably at least 60% by weight, most preferably at least 80% by weight of the particles are of particle size in a range of 1 to 200 microns and less than 20% by weight, preferably less than 15% by weight, most preferably less than 10% by weight of the particles are of particle size larger than 500 microns, with the maximum size of such particles preferably being no more than 2000 microns and, more preferably, no more than 1000 microns. In one or more embodiments, no more than 30% by weight, preferably no more than 15% by weight of the solid cleansing surfactant particles are greater than 300 microns in size.
Particle sizes greater than 200 microns can be perceived as "gritty"; accordingly, in one or more embodiments it is preferred that at least 95% by weight, more preferably at least 97% by weight, even more preferably at least 99% by weight of the solid cleansing surfactant particles are of size of not more than 200 microns. Conversely particle sizes of less than 5 microns can require specialized handling. In one or more embodiments it is preferred that at least 97% by weight, more preferably at least 99% by weight, even more preferably essentially all, of the solid surfactant particles are of size within a range of 5 to 200 microns, more particularly 5 to 150 microns and, even more particularly, 10 to 120 microns.
The solid cleansing surfactants suitable for use herein can be supplied as free flowing powders, flakes, granules or as a pre-dispersion of the surfactant particles in carrier oil. To minimize the handling issues associated with the use of fine powders, especially on a large or production scale, it is especially desirable to employ the powders as a pre-dispersion in carrier oil, preferably with a particle size/size distribution as described above. Surfactant obtained as granules or flakes are preferably premixed with carrier oil followed by high shear mixing to grind or mill the surfactant granules or flakes to the desired size/size distribution.
Together, the carrier oil, fatty acid soap crystals and solid synthetic surfactant preferably comprise 70 to 100% by weight, more particularly 80 to 98% by weight, of the subject compositions.
Desirably, the compositions of this invention are formulated such that the amount of water, if present, is not more than 5% by weight, and preferably is not more than 4% by weight, based on the total weight of the composition, with water contents of less than 5% by weight, more particularly 0.1 to 4% by weight, and, even more particularly, 0.2 to 3% by weight, based on the total weight of the composition, being of particular interest. As described in U.S. Application Serial No. 61/861,001, filed August 1, 2013, the presence of a small amount of water may give rise to aggregation of the solid synthetic surfactant, which, in turn, can aid in composition structuring. In one or more embodiments of interest, water is present in the compositions of this invention in an amount of 0.5 to 3% by weight, based on the total weight of the composition. When present, the water is preferably deionized.

Water soluble polymer is an optional ingredient that, in one or more embodiments, is highly preferred to be included in the subject compositions. Commonly, the water soluble polymers have a solubility in water of at least 1% at 25°C. These polymers can be cationic, anionic, amphoteric and/or nonionic types. They are known to enhance in-use and after-use skin/hair sensory feels, and to enhance lather creaminess, lather lubricity and/or lather stability. Water soluble cationic polymers are also known to enhance oil deposition onto the skin or hair. To provide the enhanced in-use and after-use benefits from the composition, polymers that are readily soluble upon contact with water in use, as for example, when the composition is lathered, are particularly useful for the oil continuous composition of this invention.

The amount of the water soluble polymer, when present, is typically 0.005 to 5% by weight, more particularly 0.01 to 3% by weight, based on the total weight of the composition. The polymers can be added into the composition of this invention as fine powders or, subject to the total water content parameters noted above, as aqueous solutions. The water soluble polymers are generally of relatively high molecular weight.

The average molecular weight of polymeric materials such as water soluble polymers can be determined by any of a variety of procedures, as appropriate to the particular type of polymer. For example, rheological measurements can be used to obtain viscosity average molecular weights and gel permeation chromatography or light scatter methods can be used to obtain number average molecular weights. The average molecular weights reported by suppliers of such polymers is typically higher than 10,000 Daltons, and frequently is at least 50,000 Daltons and, more particularly, at least 100,000 Daltons, with the means for measuring same and basis on which average molecular weight is reported, for example, number average molecular weight, viscosity average molecular weight, or weight average molecular weight, being subject to variation. Commonly, the polymers of interest have a number average molecular weight that is at least 50,000 Daltons and, more particularly is at least 100,000 Daltons, and frequently is less than 5,000,000 Daltons.

Examples of water soluble polymers include high molecular weight polyethylene glycols such as Polyox^{®} WSR-205 (PEG 14M), Polyox^{®} WSR-N-60K (PEG 45M), and Polyox^{®} WSR-301 (PEG 90M); the carbohydrate gums such as cellulose gum. hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, and xanthan gum; modified starch granules and pre-gelatinized cold water soluble starch; cationic polymer such as modified polysaccharides including cationic guar available from Rhodia under the trade name Jaguar^{®}; cationic modified cellulose such as UCARE Polymer JR 30 or JR 40 from Amerchol; N-Hance^{®} 3000, N-Hance^{®} 3196, N-Hance^{®} GPX 215 or N-Hance^{®} GPX 196 from Hercules; synthetic cationic polymers such as Merquat^{®} 100, Merquat^{®} 280, Merquat^{®} 281 and Merquat^{®} 550 sold by Nalco. The water soluble polymers may be used individually or as combinations of two or more polymers from the same or different classes. High molecular weight polyethylene glycols Polyox^{®} WSR-301 (PEG 90M) and Polyox^{®} WSR-N-60K (PEG 45M) and guar derivatives such as Jaguar^{®} S, Jaguar^{®} C17, and Jaguar^{®} C13, and synthetic cationic polymers such as Merquat^{®} 100 are particularly desired.

Optionally, the compositions of this invention may further comprise one or more additional ingredients. Non-limiting examples of such additional ingredients are, for example, colorants, pigments, opacifiers, fragrance (whether encapsulated or present as free-fragrance), emotive oils, vitamins and vitamin derivatives, abrasives, optical agents (including for example, reflective particles and interference pigments), pH adjusters, plant extracts, essential oils, preservatives, antioxidants, antimicrobials, viscosity modifiers, humectants, beard wetting agents, sensory agents, and skin and/or hair benefit agents (e.g., aloe, allantoin, panthenol, alpha-hydroxy acids, phospholipids, botanical oils, and amino acids to name a few). The selection and amount of any individual additional ingredient depends upon factors that include the particular ingredient, the properties desired, and the intended use of the composition in which it is employed. For example, fragrance is typically employed in an amount of 0.1 to 3.0% by weight of the composition, or higher. For many compositions, the total amount of such additional ingredients is 0.01 to 30% by weight, more particularly, 0.1 to 15% by weight, even more particularly, 1 to 10% by weight, based on the total weight of the composition. In one or more embodiments, the total amount of such additional optional ingredients is 0.5 to 5% by weight.

The compositions are conveniently prepared by dispersing the solid cleansing surfactant in carrier oil and mixing the resulting dispersion, under shear conditions, preferably under high shear, until the desired size and size distribution of solid cleansing surfactant particles is achieved, adding the fatty acid soap crystals and other additional components prior to, during and/or after formation of the dispersion of solid cleansing surfactant in carrier oil. Mixing can be carried out with little or no applied heat, with mixing at temperatures that nominally (i.e., without considering heat imparted to the mixture by shear) are ambient being preferred. Commonly such temperatures are in the range of 20 to 25°C. Additional components may be added prior to, during and/or after shear mixing, as appropriate; for example, volatile or shear sensitive components are typically added toward the completion of and/or after shear mixing to minimize their degradation or loss. The fatty acid soap crystals are preferably added as a pre-formed dispersion of the crystals in a dispersion medium. The dispersion medium desirably comprises one or more carrier oils as previously described. If desired, the pre-formed dispersion of such crystals may be made using fatty acid as a starting material by forming a solution or mixture of molten acid in the dispersion medium, adding neutralizing agent, preferably in an amount sufficient to provide a fatty acid soap having a degree of neutralization of 70 to 100%, and cooling to generate crystals of the fatty acid soap in the dispersion medium.

In a further embodiment, the subject invention relates to a method of forming a foamable, fluid personal care composition comprising a continuous oil phase in which is dispersed fatty acid soap crystals and solid synthetic cleansing surfactant and, more particularly, a foamable, fluid personal care composition according to the subject invention, the method comprising the steps of:
a) forming a dispersion of fatty acid soap crystals in a dispersion medium for the crystals;
b) dispersing solid cleansing surfactant in carrier oil to form a dispersion of solid cleansing surfactant particles, preferably at temperature within a range of from 20 to 25°C, and preferably under conditions of high shear (preferably, the dispersed solid cleansing surfactant particles are of size within a range of from 0.5 to 2000µm, at least 50% by weight of the particles are less than 200µm in size, and at least 30% by weight of the particles are less than 150 µm in size);
c) combining the dispersion of fatty acid soap crystals with the solid cleansing surfactant and carrier oil before, during and/or after the dispersion of solid cleansing surfactant particles is formed, and mixing, as needed, to form the continuous oil phase in which is dispersed fatty acid soap crystals and solid synthetic cleansing surfactant, preferably at temperature within a range of from 20 to 25°C;
wherein the composition preferably comprises:
i. 35 to 90% by weight and, more particularly, 40 to 80% by weight of carrier oil;
ii. 0.5 to10% by weight, and, more particularly, 1 to 8% by weight of fatty acid soap crystals;
iii. 10 to 65% by weight and, more particularly, 20 to 60% by weight of solid synthetic cleansing surfactant.

The compositions may be formulated as any of a variety of different personal care products including, for example, body washes, hand cleansers, facial cleansers, make-up removers, shaving compositions, and the like. In use, the compositions are compositions are diluted with water, commonly in a weight ratio of composition to water of 1:1 to 1:20, and worked into a lather before, during or after application, optionally with the aid of a sponge, pouf, cloth, brush or the like. After being used for their intended application, be it as a cleanser, shaving composition, make-up remover, or the like, the residual diluted product is removed, commonly by rinsing with water.
The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above. In specifying any range of concentration or amount, any particular upper concentration or amount can be associated with any particular lower concentration or amount. Reported ranges are inclusive of their endpoints. Unless otherwise indicated, melting points referenced herein are at ambient pressure, i.e., 1 atmosphere. (101,325 Pa). Except in the operating and comparative examples, or where otherwise explicitly indicated, all numbers in this description indicating amounts, parts, percentages, ratios and proportions of material, and conditions of reaction ought to be understood as modified by the word "about".
The following examples will more fully illustrate the embodiments of this invention. The Examples are not intended to limit the scope of the invention in any manner. All parts, percentages and proportions referred to herein and in the appended claims are by weight unless otherwise indicated.

### Examples

In the Examples that follow, stability was determined as a function of oil separation, employing an approximately 100g sample that was introduced to a 4oz (118ml) cylindrical glass jar having an inner diameter of 1.8 inches (4.6cm). If a sample was not stable, disruption of the oil phase and separation of the oil/surfactant dispersion occurred, resulting in a layer of oil on top of the sample. The height of the oil layer was measured and divided by the total height of the sample and reported as the "% oil layer separation". A 0% oil layer separation means the sample was stable without any visible phase separation of surfactant particle from the oil.

Viscosity was measured using a standard AR-G2 stress-controlled rheometer from Texas instruments (or equivalent) and carrying out steady shear rate sweep measurements from 0.01 to 100s⁻¹, using a 40mm cone and plate geometry, with a cone angle of 2° and a sample gap of 61 microns and collecting three sample points per decade, with all the measurements being done at constant temperature of 23°C.

### Examples 1 to 10

Sodium lauroyl isethionate (SLI) powder (ex Yongan Daily Chemical Co.) was used to prepare a series of cleansing composition formulations as described in Table 1. The neutralized fatty acid dispersions reported as ingredients were prepared by combining 47.4 parts of the indicated oil, 21.6 parts of lauric acid, 10.8 parts of myristic acid and 3.6 parts stearic acid in a vessel equipped with a 3-blade overhead mixer (IKA) and heating the resulting oil/acid mixture to 70-75°C, with stirring; when all the fatty acids had dissolved (as indicated by the formation of a clear solution); 16.6 parts of a solution of 45 wt% KOH in water was slowly added to the fatty/acid oil solution over a period of 5 to 10 minutes to neutralize the fatty acids; after mixing for an additional 5 minutes at 70°C, the mixture was cooled to ambient temperature, yielding a dispersion of fine soap crystals in oil.

The Table 1 formulations were prepared by first adding the indicated oil component to a glass jar equipped with an overhead 3-blade mixer. The water soluble polymer powders (i.e. Polyox™ WSR-301(PEG-90M), Polyox™ WSR N-60K (PEG-45M) or Jaguar^{®} S guar gum) and perfume were added and mixed with the oil. The surfactant was then gradually added over a period of 5 to 10 minutes while mixing at room temperature (20 to 25°C) to uniformly disperse the solid surfactant particles in the oil. After addition of the surfactant was complete, the remaining ingredients were added and the mixture was mixed at high speed (1000 to 1500 rpm) for another 3 to 6 minutes. The formulations were aged at room temperature for seven days and then evaluated for product stability and viscosity, the results of which are reported in Table 1.

**TABLE 1**

| | **1** | **2** | **3** | **4** | **5** | **6** | **7 (Comp)** | **8 (Comp)** | **9 (Comp)** | **10 (Comp)** |
|---|---|---|---|---|---|---|---|---|---|---|
| **Ingredient (wt.%)** | | | | | | | | | | |
| White Mineral oil (Drakeol® 7 Lt. Min. Oil, ex Penreco) | 42.10 | 48.50 | - | - | - | - | 48.50 | - | - | - |
| Mineral Oil (Hydrobrite® 1000, ex Sonneborn) | - | - | - | - | 63.45 | 73.45 | - | - | - | 58.80 |
| Soybean oil | - | - | 53.55 | - | - | - | - | 58.85 | - | - |
| Silicone Fluid (Dimethicone; 350 cps) | - | - | - | 53.45 | - | - | | | 68.85 | |
| Na lauroyl isethionate powder (ex. Yongan Daily Chemical Co.) | 50.00 | 40.00 | 34.65 | 30.00 | 20.00 | 10.00 | 50.00 | 40.00 | 30.00 | 40.00 |
| Guar Gum; CAS No. 9000-30-0 (Jaguar® S ex. Rhodia) | 0.50 | 0.50 | - | - | | | 0.50 | - | - | - |
| PEG-45M (PolyoxTM WSR N-60K Water Soluble Resin ex. Dow Chemical) | - | - | 0.06 | - | | | - | - | - | - |
| PEG-90M (PolyoxTM WSR-301Water Soluble Resin ex. Dow Chemical) | - | - | - | 0.15 | 0.15 | 0.15 | - | 0.15 | 0.15 | 0.20 |
| Citric acid powder | 0.40 | - | 0.84 | 0.40 | 0.40 | 0.40 | - | - | - | - |
| Perfume | 1.00 | 1.00 | 1.00 | 1.00 | 1.00 | 1.00 | 1.00 | 1.00 | 1.00 | 1.00 |
| Neutralized tatty acid dispersion in Drakeol® 7 Lt. Min. Oil (Soap Dispersion A) | 6.00 | 10.00 | - | 15.00 | 15.00 | 15.00 | - | - | - | - |
| Neutralized tatty acid dispersion in soybean oil (Soap Dispersion B) | - | - | 9.90 | - | - | - | - | - | - | - |
| Total tree water content (From water- containing ingredients) | 0.7 | 1.2 | 1.2 | 1.7 | 1.7 | 1.7 | 0.0 | 0.0 | 0.0 | 0.0 |
| % clear oil layer separation | <2% | 0% | 0% | 0% | 0% | 0% | 27.4% | 25.8% | 23.3% | 12.2% |
| Viscosity at 0.01 sec-1 (kg**•**m-1) | 6061 | 6148 | 14090 | 12650 | 1855 | 922 | 291 | 11 | 9 | 22 |

As demonstrated by the Table 1 data, the compositions that included neutralized fatty acid crystals (from Soap Dispersion A or Soap Dispersion B) had increased viscosity and enhanced stability compared to comparative Examples 7 to 10.

### Examples 11 to 18

The effect of surfactant particle size on stability was evaluated in compositions having a relatively low content of fatty acid soap. In this series of Examples, sodium cocoyl isethionate (SCI) in prill form (Jordapon^{®} Cl prill, ex BASF) was pulverized and fractionated using a series of U.S.A. Standard Sieves. Particles having a size in the range of <2000 to 500 µm; <500 to 300 µm; <300 to 180µm; <180 to 75µm, <75 to 45µm; and less than 45µm were collected separately for sample preparation. For a reported fraction, the particles passed through a sieve having a mesh size corresponding to the highest value in the range and were held on a sieve having a mesh size corresponding to the lowest value in the range. For example, particles in the range of <2000 to 500µm passed through a sieve having a mesh size of 2000µmand were held on a sieve having a mesh size of 500µm.

Following the procedure described in the foregoing Examples, formulations as described in Table 2 were prepared and the stability thereof was measured.

**Table 2**

| | **Sodium Cocoyl isethionate Fractions** | | | | | | | |
|---|---|---|---|---|---|---|---|---|
| | FR1 | FR2 | FR3 | FR4 | FR5 | FR6 | FR7 | FR8 |
| | wt% ot surfactant particle tractionate | | | | | | | |
| Particle size range <2000-500 µm | 100% | - | - | - | - | 40% | 20% | 50% |
| Particle size range <500-300 µm | - | 100% | **-** | - | - | 25% | 10% | 25% |
| Particle size range <300-180 µm | - | - | 100% | - | - | - | - | - |
| Particle size range <180-75 µm range | - | - | - | 100% | - | 15% | 20% | 5% |
| Particle size range <75-45 µm | - | - | - | - | 100% | 20% | 25% | 10% |
| Particle size < 45 µm | - | - | - | - | - | - | - | - |
| | | | | | | | | |

| | **Example** | | | | | | | |
|---|---|---|---|---|---|---|---|---|
| | **11 (Comp)** | **12 (Comp)** | **13 (Comp)** | **14** | **15** | **16** (Comp) | **17** | **18 (Comp)** |
| **Ingredient (wt. %)** | | | | | | | | |
| Total amount of sodium cocoyl isethionate; fraction as described above | 50.00 FR1 | 50.00 FR2 | 50.00 FR3 | 50.00 FR4 | 50.00 FR5 | 50.00 FR6 | 50.00 FR7 | 50.00 FR8 |
| White Mineral oil (Drakeol® 7 Lt. Min. Oil, ex Penreco) | 41.15 | 41.15 | 41.15 | 41.15 | 41.15 | 41.15 | 41.15 | 41.15 |
| PEG-90M (PolyoxTM WSR-301 Water Soluble Resin ex. Dow Chemical) | 0.45 | 0.45 | 0.45 | 0.45 | 0.45 | 0.45 | 0.45 | 0.45 |
| Neutralized tatty acid dispersion in Drakeol® 7 Lt. Min. Oil (Soap Dispersion A) | 6.00 | 6.00 | 6.00 | 6.00 | 6.00 | 6.00 | 6.00 | 6.00 |
| Tego® Betain F (28wt.% Na cocoyl aminopropyl betaine in water) | 2.00 | 2.00 | 2.00 | 2.00 | 2.00 | 2.00 | 2.00 | 2.00 |
| Citric acid powder | 0.40 | 0.40 | 0.40 | 0.40 | 0.40 | 0.40 | 0.40 | 0.40 |
| % clear oil layer separation | 16.7% | 14.8% | 8.3% | 0% | 0% | 0% | 0% | 8.7% |

As demonstrated by the Table 2 data, the size/size distribution of the surfactant particles was found to impact composition stability in this formulation.

### Examples 19 to 31

Examples 19 to 31 show the effect of soap neutralization on viscosity in various oil/surfactant compositions.

The fatty acid soap dispersions used in these Examples were prepared according to the formulations given in Table 3 by combining the indicated fatty acids and mineral oil in a glass jar equipped with a 3-blade overhead mixer, and heating the resulting oil/acid mixture to 70-75°C, with stirring; when all the fatty acids had dissolved (as indicated by the formation of a clear solution), the indicated base was slowly added to neutralize the fatty acid; after adding all the base, the mixture was cooled to ambient temperature, yielding a dispersion of fine soap crystals in oil.

**Table 3**

| | **Soap Dispersion** | | | | | | | | |
|---|---|---|---|---|---|---|---|---|---|
| **Ingredient (wt.%)** | **C** | **D** | **E** | **F** | **G** | **H** | **I** | **J** | **K** |
| **Fatty Acid** | | | | | | | | | |
| Lauric acid | 12.00 | 12.00 | 12.00 | 12.00 | 10.00 | 20.00 | 20.00 | 20.00 | 20.00 |
| Myristic acid | 6.00 | 6.00 | 6.00 | 6.00 | 6.00 | -- | -- | -- | -- |
| Palmitic acid | 1.00 | 1.00 | 1.00 | 1.00 | 2.00 | -- | -- | -- | -- |
| Stearic acid | 1.00 | 1.00 | 1.00 | 1.00 | 2.00 | -- | -- | -- | -- |
| | | | | | | | | | |
| Oil | | | | | | | | | |
| (Hydrobrite® 1000 ex Sonneborn) | 73.07 | 73.85 | 70.75 | 68.90 | 67.80 | 67.80 | 65.70 | 70.70 | 69.09 |
| | | | | | | | | | |
| Base | | | | | | | | | |
| 45% wt.% KOH in Water | -- | -- | 9.25 | 11.10 | -- | -- | -- | | |
| 50wt.% NaOH in water | 6.93 | 6.15 | -- | -- | -- | -- | -- | 3.20 | 3.76 |
| Triethanol amine | -- | -- | -- | -- | 12.20 | 12.20 | 14.30 | 6.10 | 7.15 |
| **Degree of neutralization** | 93.3% | 82.7% | 80% | 96.4% | 90% | 80% | 9b% | 80% | 9b% |

The surfactant-in-oil premixes referred to in Tables 4 and 5 were prepared to the specified loadings using a BlendTec™ Model ICB7 high speed mixer. In preparing the premixes, the surfactant powder and mineral oil (Hydrobrite^{®} 1000, ex Sonnebom) were added to the blender jar and mixed 4 times (for 50 seconds each time), at Blendtec preset #3 speed cycle.

The formulations of Examples 19 to 31 are given in Tables 4 and 5. The formulations were prepared using an IKA M20 S3 mixer. The surfactant-in-oil premix, soap dispersion, and all the other composition ingredients were added to the mixing chamber, mixed by hand for 2 to 3 minutes using a spatula, and then twice blended (for 25 to 30 seconds each time) using the mixer. After aging the resulting formulations at ambient temperature overnight, viscosity measurements were taken using the procedure described above. The results are reported in Tables 4 and 5.

**Table 4**

| | **EXAMPLE** | | | | | | | |
|---|---|---|---|---|---|---|---|---|
| **Ingredient** (**wt.%)** | **19 (Comp)** | **20** | **21** | **22** | **23** | **24** | **25** | **26** |
| Mineral Oil (Hydrobrite® 1000, ex Sonnebom) | 26.80 | 19.30 | 11.80 | 4.30 | - | 1.70 | 1.60 | 1.70 |
| Isopropyl palmitate | - | - | - | - | 11.80 | - | - | - |
| Betaine Premix 25wt%Tego® Betain CK D, ex Degussa, in mineral oil | 20.00 | 20.00 | 20.00 | 20.00 | 20.00 | 30.00 | 30.00 | 30.00 |
| SLG Premix 25 wt% sodium lauroyl glycinate (Galsoft TM SLG powder ex Galaxy) in mineral oil | 40.00 | 40.00 | 40.00 | 40.00 | 40.00 | 40.00 | 40.00 | 40.00 |
| SLI Premix 50 wt% sodium lauroyl isethionate (SLI powder, ex Yongan Daily Chemical Company) in mineral oil) | 10.00 | 10.00 | 10.00 | 10.00 | 10.00 | 10.00 | 10.00 | 10.00 |
| MerquatTM 100 Polymer Polyquaternium -6 (ex. Nalco) | 0.50 | 0.50 | 0.50 | 0.50 | 0.50 | 0.50 | 0.60 | 0.50 |
| Citric acid powder | 1.00 | 1.00 | 1.00 | 1.00 | 1.00 | 1.10 | 1.10 | 1.10 |
| Abil® EM90 Cetyl PEG/PPG-10/1 Dimethicone | 0.50 | 0.50 | 0.50 | 0.50 | 0.50 | 0.50 | 0.50 | 0.50 |
| Soap Dispersion** | - | 7.50 C | 15.00 C | 22.50 C | 15.00 C | 15.00 E | 15.00 F | 15.00 D |
| PEG90M PolyoxTM WSR-301 Water Soluble Resin (ex. Dow Chemical) | 0.20 | 0.20 | 0.2 | 0.20 | 0.20 | 0.20 | 0.20 | 0.20 |
| Perfume | 1.00 | 1.00 | 1.00 | 1.00 | 1.00 | 1.00 | 1.00 | 1.00 |
| | | | | | | | | |

| **Viscosity** | | | | | | | | |
|---|---|---|---|---|---|---|---|---|
| Viscosity at 0.01sec-1 (kg•m-1) | 245 | 1326 | 3429 | 4916 | 2232 | 3154 | 3850 | 3939 |
| Viscosity at 1 sec-1 (kg•m-1) | 8.4 | 24.1 | 60.4 | 85.3 | 43.8 | 81.9 | 68.8 | 87.7 |

| | | | | | | | | |
|---|---|---|---|---|---|---|---|---|
| *Tego^{®}Betain CK D (coamidopropyl betaine) is provided as a powder that is reported to contain >80% active. **See Table 3. | | | | | | | | |

As demonstrated by the Table 4 data, viscosity tended to increase with soap concentration. Desirable increases in viscosity were obtained over the reported range of fatty acid soap neutralization, i.e., 80 to 96.4%.

**Table 5**

| | Example | | | | |
|---|---|---|---|---|---|
| | 27 | 28 | 29 | 30 | 31 |
| Ingredient (wt.%) | | | | | |
| Mineral Oil (Hydrobrite® 1000, exSonnebom) | 11.70 | 11.70 | 11.70 | 11.70 | 11.70 |
| Betaine Premix (25wt%Tego® Betain CK Din mineral oil) | 20.00 | 20.00 | 20.00 | 20.00 | 20.00 |
| SLG Premix (25 wt% SLG in mineral oil) | 40.00 | 40.00 | 40.00 | 40.00 | 40.00 |
| SLI Premix (50 wt% SLI in mineral oil) | 10.00 | 10.00 | 10.00 | 10.00 | 10.00 |
| MerquatTM 100 Polymer Polyquaternium -6 (ex. Nalco) | 0.50 | 0.50 | 0.50 | 0.50 | 0.50 |
| Citric acid powder | 1.10 | 1.10 | 1.10 | 1.10 | 1.10 |
| Abil® EM90 Cetyl PEG/PPG-10/1 Dimethicone | 0.50 | 0.50 | 0.50 | 0.50 | 0.50 |
| Soap dispersion* | 15.00 G | 15.00 H | 15.00 I | 15.00 J | 15.00 K |
| PEG90M PolyoxTM WSR-301 Water Soluble Resin ex. Dow Chemical) | 0.20 | 0.20 | 0.20 | 0.20 | 0.20 |
| Perfume | 1.00 | 1.00 | 1.00 | 1.0 | 1.00 |
| Viscosity | | | | | |
| Viscosity at 0.01 sec-1 (kg•m-1) | 5217 | 16970 | 17110 | 3208 | 2821 |
| Viscosity at 1 sec-1 (kg•m-1) | 155.8 | 342.3 | 388 | 59.16 | 56.2 |

| | | | | | |
|---|---|---|---|---|---|
| *See Table 3. | | | | | |

The Table 5 Examples demonstrate the effectiveness of a wide range of neutralized fatty acids at thickening oil continuous formulations.

## Claims

1. A foamable, fluid personal care composition comprising a continuous oil phase in which is dispersed fatty acid soap crystals and solid synthetic cleansing surfactant wherein the composition comprises:
i. 35 to 90% by weight of carrier oil;
ii. 0.5 to10% by weight of fatty acid soap crystals, wherein fatty acid soap means salt of aliphatic alkane or alkene monocarboxylic fatty acids having 6 to 22 carbon atoms; and
iii. 10 to 65% by weight of solid synthetic cleansing surfactant wherein the solid synthetic cleansing surfactant is present in the form of particles of size within a range of from 0.5 to 2000µm, wherein at least 50% by weight of the particles are less than 200µm in size and at least 30% by weight of the particles are less than 150 µm in size.

2. The composition according to claim 1 wherein, together, the carrier oil, fatty acid soap crystals, and solid synthetic cleansing surfactant constitute 70 to 100% of the total weight of the composition.

3. The composition according to claim 1 or claim 2 wherein the carrier oil includes one or more oils selected from hydrocarbon oils, ether oils, ester oils, fatty alcohols, and silicone oils.

4. The composition according to any one of claims 1 to 3 wherein 90 to 100% by weight of the carrier oil is one or more oils selected from non-volatile hydrocarbon oils, triglyceride oils, and silicone oils.

5. The composition according to any one of the preceding claims wherein the carrier oil comprises at least 95% by weight of non-volatile oil.

6. The composition according to any one of the preceding claims further comprising up to 5% by weight, based on the total weight of the composition, of water.

7. The composition according to any one of the preceding claims further comprising water soluble polymer.

8. The composition according to claim 7 wherein water soluble polymer is present in an amount of 0.01 to 3% by weight, based on the total weight of the composition.

9. The composition according to any one of the preceding claims wherein 90 to 100% by weight of the solid synthetic cleansing surfactant is one or more surfactants selected from anionic and amphoteric surfactants.

10. The composition according to any one of the preceding claims wherein no more than 30% by weight of the solid synthetic cleansing surfactant particles are greater than 500µm.

11. The composition according to any one of the preceding claims wherein the solid synthetic cleansing surfactant comprises sodium lauroyl isethionate.

12. The composition according to any one of claims 1 to 11 wherein water is present in an amount of 0.1 to 4% by weight, based on the total weight of the composition.

13. A method of forming a foamable, fluid personal care composition comprising a continuous oil phase in which is dispersed fatty acid soap crystals and solid synthetic cleansing surfactant, the method comprises the steps of:
a) forming a dispersion of fatty acid soap crystals in a dispersion medium for the crystals;
b) mixing solid synthetic cleansing surfactant with carrier oil to form a dispersion of solid synthetic cleansing surfactant particles in carrier oil,
c) combining the dispersion of fatty acid soap crystals with the solid synthetic cleansing surfactant and carrier oil before, during and/or after the dispersion of solid cleansing surfactant particles is formed, and mixing, as needed, to form the continuous oil phase in which is dispersed fatty acid soap crystals and solid synthetic cleansing surfactant;
wherein the composition comprises:
i. 35 to 90% by weight of carrier oil;
ii. 0.5 to10% by weight of fatty acid soap crystals; and
iii. 10 to 65% by weight of solid synthetic cleansing surfactant, wherein the solid synthetic cleansing surfactant is present in the form of particles of size within a range of from 0.5 to 2000µm, wherein at least 50% by weight of the particles are less than 200µm in size and at least 30% by weight of the particles are less than 150 µm in size; and
wherein fatty acid soap means salt of aliphatic alkane or alkene monocarboxylic fatty acids having 6 to 22 carbon atoms.

14. The method of claim 13 wherein mixing of the carrier oil and solid synthetic surfactant is carried out at temperature within a range of from 20 to 25°C.

## Patentansprüche

1. Schäumbare flüssige Körperpflegezusammensetzung, umfassend eine kontinuierliche Ölphase, in der Fettsäureseifenkristalle und festes synthetisches Reinigungstensid dispergiert sind, wobei die Zusammensetzung umfasst:
i. 35 bis 90 Gewichts-% Trägeröl,
ii. 0,5 bis 10 Gewichts-% Fettsäureseifenkristalle, wobei Fettsäureseife das Salz von aliphatischen Alkan- oder Alken-monocarbonfettsäuren mit 6 bis 22 Kohlenstoffatomen bedeutet, und
iii. 10 bis 65 Gewichts-% festes synthetisches Reinigungstensid, wobei das feste synthetische Reinigungstensid in Form von Partikeln einer Größe innerhalb eines Bereichs von 0,5 bis 2000 µm vorliegt, wobei mindestens 50 Gewichts-% der Partikel weniger als 200 µm und mindestens 30 Gewichts-% der Partikel weniger als 150 µm groß sind.

2. Zusammensetzung nach Anspruch 1, wobei, zusammen, das Trägeröl, die Fettsäureseifenkristalle und das feste synthetische Reinigungstensid 70 bis 100% des Gesamtgewichts der Zusammensetzung ausmachen.

3. Zusammensetzung nach Anspruch 1 oder Anspruch 2, wobei das Trägeröl ein oder mehrere Öle einbezieht, die unter Kohlenwasserstoffölen, Etherölen, Esterölen, Fettalkoholen und Silikonölen ausgewählt sind.

4. Zusammensetzung nach irgendeinem der Ansprüche 1 bis 3, wobei 90 bis 100 Gewichts-% des Trägeröls ein oder mehrere Öle darstellen, die unter nichtflüchtigen Kohlenwasserstoffölen, Triglyceridölen und Silikonölen ausgewählt sind.

5. Zusammensetzung nach irgendeinem der vorhergehenden Ansprüche, wobei das Trägeröl mindestens 95 Gewichts-% nichtflüchtiges Öl umfasst.

6. Zusammensetzung nach irgendeinem der vorhergehenden Ansprüche, die des Weiteren bis zu 5 Gewichts-%, bezogen auf das Gesamtgewicht der Zusammensetzung, Wasser umfasst.

7. Zusammensetzung nach irgendeinem der vorhergehenden Ansprüche, die des Weiteren wasserlösliches Polymer umfasst.

8. Zusammensetzung nach Anspruch 7, wobei das wasserlösliche Polymer in einer Menge von 0,01 bis 3 Gewichts-%, bezogen auf das Gesamtgewicht der Zusammensetzung, vorliegt.

9. Zusammensetzung nach irgendeinem der vorhergehenden Ansprüche, wobei 90 bis 100 Gewichts-% des festen synthetischen Reinigungstensids ein oder mehrere Tenside darstellen, die unter anionischen und amphoteren Tensiden ausgewählt sind.

10. Zusammensetzung nach irgendeinem der vorhergehenden Ansprüche, wobei nicht mehr als 30 Gewichts-% der Partikel des festen synthetischen Reinigungstensids größer als 500 µm sind.

11. Zusammensetzung nach irgendeinem der vorhergehenden Ansprüche, wobei das feste synthetische Reinigungstensid Natriumlauroylisethionat umfasst.

12. Zusammensetzung nach irgendeinem der Ansprüche 1 bis 11, wobei Wasser in einer Menge von 0,1 bis 4 Gewichts-%, bezogen auf das Gesamtgewicht der Zusammensetzung, vorliegt.

13. Verfahren zur Ausbildung einer schäumbaren flüssigen Körperpflegezusammensetzung, umfassend eine kontinuierliche Ölphase, in der Fettsäureseifenkristalle und festes synthetisches Reinigungstensid dispergiert sind, wobei das Verfahren die Schritte umfasst:
a) Bilden einer Dispersion der Fettsäureseifenkristalle in einem Dispersionsmedium für Kristalle,
b) Mischen von festem synthetischen Reinigungstensid mit Trägeröl, um eine Dispersion von Partikeln festen synthetischen Reinigungstensids im Trägeröl zu bilden,
c) Kombinieren der Dispersion der Fettsäureseifenkristalle mit dem festen synthetischen Reinigungstensid und dem Trägeröl, bevor, während und/oder nachdem die Dispersion der Partikel des festen Reinigungstensids gebildet ist, und Mischen, bei Bedarf, um die kontinuierliche Ölphase zu bilden, in der die Fettsäureseifenkristalle und das feste synthetische Reinigungstensid dispergiert sind,
wobei die Zusammensetzung umfasst:
i. 35 bis 90 Gewichts-% Trägeröl,
ii. 0,5 bis 10 Gewichts-% Fettsäureseifenkristalle und
iii. 10 bis 65 Gewichts-% festes synthetisches Reinigungstensid, wobei das feste synthetische Reinigungstensid in Form von Partikeln einer Größe innerhalb eines Bereichs von 0,5 bis 2000 µm vorliegt, wobei mindestens 50 Gewichts-% der Partikel weniger als 200 µm und mindestens 30 Gewichts-% der Partikel weniger als 150 µm groß sind, und
wobei die Fettsäureseife das Salz von aliphatischen Alkan- oder Alkenmonocarbonfettsäuren mit 6 bis 22 Kohlenstoffatomen bedeutet.

14. Verfahren nach Anspruch 13, wobei das Mischen des Trägeröls und des festen synthetischen Tensids bei einer Temperatur innerhalb des Bereichs von 20 bis 25°C durchgeführt wird.

## Revendications

1. Composition pour le soin de la personne fluide, apte à mousser comprenant une phase d'huile continue dans laquelle des cristaux de savon d'acide gras et un tensioactif nettoyant synthétique solide sont dispersés dans laquelle la composition comprend :
i. de 35 à 90 % en masse d'huile de support ;
ii. de 0,5 à 10 % en masse de cristaux de savon d'acide gras, dans laquelle ledit savon d'acide gras indique un sel d'acides gras alcane ou alcène monocarboxyliques aliphatiques ayant de 6 à 22 atomes de carbone ; et
iii. de 10 à 65 % en masse de tensioactif nettoyant synthétique solide dans laquelle le tensioactif nettoyant synthétique solide est présent dans la forme de particules de taille dans un intervalle de 0,5 à 2 000 µm, dans laquelle au moins 50 % en masse des particules sont de taille inférieure à 200 µm et au moins 30 % en masse des particules sont de taille inférieure à 150 µm.

2. Composition selon la revendication 1 dans laquelle, ensemble, les huiles de support, cristaux de savon d'acide gras et tensioactif nettoyant synthétique solide constituent de 70 à 100 % de la masse totale de la composition.

3. Composition selon la revendication 1 ou la revendication 2, dans laquelle l'huile de support comprend une ou plusieurs huiles choisies parmi des huiles hydrocarbonées, des huiles d'éther, des huiles d'ester, des alcools gras, et des huiles de silicone.

4. Composition selon l'une quelconque des revendications 1 à 3, dans laquelle de 90 à 100 % en masse de l'huile de support sont une ou plusieurs huiles choisies parmi des huiles hydrocarbonées non volatiles, des huiles de triglycérides et des huiles de silicone.

5. Composition selon l'une quelconque des revendications précédentes, dans laquelle l'huile de support comprend au moins 95 % en masse d'huile non volatile.

6. Composition selon l'une quelconque des revendications précédentes comprenant de plus jusqu'à 5 % en masse, rapportée à la masse totale de la composition, d'eau.

7. Composition selon l'une quelconque des revendications précédentes comprenant de plus un polymère soluble dans l'eau.

8. Composition selon la revendication 7, dans laquelle le polymère soluble dans l'eau est présent dans une quantité de 0,01 à 3 % en masse, rapportée à la masse totale de la composition.

9. Composition selon l'une quelconque des revendications précédentes, dans laquelle de 90 à 100 % en masse du tensioactif nettoyant synthétique solide sont un ou plusieurs tensioactifs choisis parmi des tensioactifs anioniques et amphotères.

10. Composition selon l'une quelconque des revendications précédentes, dans laquelle au plus 30 % en masse des particules de tensioactif nettoyant synthétique solide sont supérieures à 500 µm.

11. Composition selon l'une quelconque des revendications précédentes, dans laquelle le tensioactif nettoyant synthétique solide comprend du lauroyliséthionate de sodium.

12. Composition selon l'une quelconque des revendications 1 à 11, dans laquelle de l'eau est présente dans une quantité de 0,1 à 4 % en masse, rapportée à la masse totale de la composition.

13. Procédé de formation d'une composition pour le soin de la personne fluide, apte à mousser comprenant une phase d'huile continue dans laquelle des cristaux de savon d'acide gras et un tensioactif nettoyant synthétique solide sont dispersés, le procédé comprend les étapes de :
a) formation d'une dispersion de cristaux de savon d'acide gras dans un milieu de dispersion pour les cristaux ;
b) mélange de tensioactif nettoyant synthétique solide avec une huile de support pour former une dispersion de particules de tensioactif nettoyant synthétique solide dans une huile de support,
c) combinaison de la dispersion de cristaux de savon d'acide gras avec le tensioactif nettoyant synthétique solide et l'huile de support avant, pendant et/ou après que la dispersion des particules de tensioactif nettoyant solide est formée, et mélange, si nécessaire, pour former la phase d'huile continue dans laquelle des cristaux de savon d'acide gras et un tensioactif nettoyant synthétique solide sont dispersés ;
dans lequel la composition comprend :
i. de 35 à 90 % en masse d'huile de support ;
ii. de 0,5 à 10 % en masse de cristaux de savon d'acide gras ; et
iii. de 10 à 65 % en masse de tensioactif nettoyant synthétique solide, dans lequel le tensioactif nettoyant synthétique solide est présent dans la forme de particules de taille dans un intervalle de 0,5 à 2 000 µm, dans lequel au moins 50 % en masse des particules sont de taille inférieure à 200 µm et au moins 30 % en masse des particules sont de taille inférieure à 150 µm ; et
dans lequel le savon d'acide gras indique un sel d'acides gras alcane ou alcène monocarboxyliques aliphatiques ayant de 6 à 22 atomes de carbone.

14. Procédé selon la revendication 13, dans lequel le mélange de l'huile de support et de tensioactif synthétique solide est réalisé à une température dans un intervalle de 20 à 25°C.
